# FASCICULE DE BREVET EUROPEEN

(11) **EP 0 629 616 B1**
(45) Date de publication et mention de la délivrance du brevet: **29.08.2001**
(21) Numéro de dépôt: 94420167.2
(22) Date de dépôt: 10.06.1994
(51) Int. Cl.: C07D 233/80, C07D 233/84, C07D 233/70, C07D 233/86, C07D 233/74, C07D 233/76, C07D 233/78, C07C 331/24, C07C 229/36, C07C 243/32, A01N 43/50

(54) **Dérivés optiquements actifs de 2-imidazoline-5-ones comme fongicides**
Optisch aktive 2-Imidazoline-5-one-Derivate als Fungizide
Optically active 2-imidazolin-5-one derivatives as fungicides

(30) Priorité: 18.06.1993 FR 9307663; 21.02.1994 FR 9402144
(43) Date de publication de la demande: 21.12.1994
(73) Titulaire: AVENTIS CROPSCIENCE S.A., 69009 Lyon (FR)
(72) Inventeur: Bascou, Jean-Philippe, F-69009 Lyon (FR); Lacroix, Guy, F-69009 Lyon (FR); Gadras, Alain, F-69009 Lyon (FR); Perez, Joseph, F-69009 Lyon (FR)

(56) Documents cités:
- EP-A- 0 283 245
- EP-A- 0 303 863
- EP-A- 0 551 048
- WO-A-93/24467
- FR-A- 1 549 758
- FR-A- 2 693 192
- GB-A- 967 167
- US-A- 4 957 933
- BANQUE DE DONNEES STN: CAS REGISTRY HANDBOOK 1965-1971 , AMERICAN CHEMICAL SOCIETY * RN: 4855-22-5, 4855-23-6, 4855-24-7, 4855-25-8, 4855-26-9, 4855-27-0 *
- BANQUE DE DONNEES STN: CAS REGISTRY HANDBOOK 1965-1971 , AMERICAN CHEMICAL SOCIETY * RN: 4892-65-3, 4892-66-4 *
- CHEMICAL ABSTRACTS, vol. 95, no. 19, 9 Novembre 1981, Columbus, Ohio, US; abstract no. 169042n, & TETRAHEDRON LETTERS, vol.22, no.26, 1981, OXFORD GB pages 2435 - 2438 A. PADWA ET AL. & CAS REGISTY HANDBOOK 1965-1971
- CHEMICAL ABSTRACTS, vol. 83, no. 5, 4 Août 1975, Columbus, Ohio, US; abstract no. 43702m, & YAKUGAKU ZASSHI, vol.95, no.1, 1975 pages 28 - 32 ISOO ITO ET AL. & CAS REGISTRY HANDBOOK 1975 SUPPL. , AMERICAN CHEMICAL SOCIETY
- CHEMICAL ABSTRACTS, vol. 85, no. 15, 11 Octobre 1976, Columbus, Ohio, US; abstract no. 108496k, & CHEMISCHE BERICHTE, vol.109, no.6, 1976, WEINHEIM DE pages 2331 - 2334 S. GOETZE ET AL. & CAS REGISTRY HANDBOOK 1976 SUPPL. , AMERICAN CHEMICAL SOCIETY
- CHEMICAL ABSTRACTS, vol. 108, no. 9, 29 Février 1988, Columbus, Ohio, US; abstract no. 75264w, & CHEMISCHE BERICHTE, vol.121, no.1, 1988, WEINHEIM DE pages 67 - 73 M. D'ANELLO ET AL. & CAS REGISTRY HANDBOOK 1987 SUPPL. , AMERICAN CHEMICAL SOCIETY
- CHEMICAL ABSTRACTS, vol. 98, no. 13, 28 Mars 1983, Columbus, Ohio, US; abstract no. 107198w, & JOURNAL OF ORGANIC CHEMISTRY, vol.48, no.5, 1983, EASTON US pages 695 - 703 A. PADWA ET AL. & CAS REGISTRY HANDBOOK 1981 SUPPL. , AMERICAN CHEMICAL SOCIETY
- JOURNAL OF THE CHEMICAL SOCIETY, PERKIN TRANSACTIONS 1, 9 Septembre 1990, LETCHWORTH GB pages 3003 - 3009 T. YAMAMOTO ET AL.
- CHEMICAL ABSTRACTS, vol. 56, no. 11, 28 Mai 1962, Columbus, Ohio, US; abstract no. 12873f, & BULLETIN DE LA SOCIETE CHIMIQUE DE FRANCE, 1961, PARIS FR pages 2161 - 2165 M. ROBBA ET AL. & CHEM. ABS. 7TH COLLECTIVE INDEX, VOL 56-65 (1962-1966) , AMERICAN CHEMICAL SOCIETY
- BANQUE DE DONNEES STN: CAS REGISTRY HANDBOOK 1965-1971 , AMERICAN CHEMICAL SOCIETY * RN: 7502-05-8 *

## Description

La présente invention a pour objet de nouveaux dérivés optiquement actifs de 2-imidazoline-5-ones à usage phytosanitaire, leur procédé de préparation et les composés éventuellement utilisables comme intermédiaires dans les procédés de préparation. Elle concerne également des compositions fongicides à base de ces composés et un procédé de traitement des maladies fongiques des cultures utilisant ces composés.

Les composés racémiques dérivés de 2-imidazoline-5-ones sont décrits dans les demandes de brevet européen EP 551048 et EP 599749, et dans les demandes internationales WO 94/01410 et WO 93/24467.

Il a à présent été découvert que l'un des isomères optiques de ces composés a une activité biologique qui est très supérieure à celle de l'autre isomère et à celle du mélange racémique.

Un but de l'invention est donc de fournir de nouveaux composés utiles pour la lutte contre les maladies fongiques des cultures.

Un autre but de l'invention est de fournir de nouveaux dérivés de 2-imidazoline-5-ones, actifs à une dose réduite par rapport à celle des dérivés racémiques.

Il a maintenant été trouvé que ces buts pouvaient être atteints grâce aux produits de l'invention, qui sont des dérivés optiquement actifs de 2- ' imidazoline-5-ones de formule générale I: 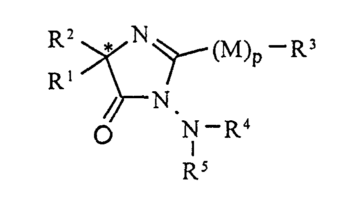 dans laquelle :
- M représente un atome d'oxygène ou de soufre, ou un radical CH₂, éventuellement halogéné ;
- p est un nombre entier égal à 0 ou 1 ;
   * signifie que l'atome de carbone asymétrique correspondant a une configuration stéréospécifique ;
- R¹ et R² sont différents et représentent :
   - un radical alkyl ou haloalkyl de 1 à 6 atomes de carbone ou
   - un radical alkoxyalkyl, alkylthioalkyl, alkylsulfonylalkyl, mono alkylaminoalkyl, alcènyl ou alcynyl de 2 à 6 atomes de carbone ou
   - un radical dialkylaminoalkyl ou cycloalkyl de 3 à 7 atomes de carbone ou
   - un radical aryl, comprenant phényl, naphtyl, thiényl, furyl, pyridyl, benzothiényl, benzofuryl, quinolinyl, isoquinolinyl, ou méthylène dioxyphényl, éventuellement substitué par 1 à 3 groupements choisis parmi R⁶ ou
   - un radical arylalkyl, aryloxyalkyl, arylthioalkyl ou arylsulfonylalkyl, les termes aryl et alkyl ayant les définitions données ci-dessus ou
   - R¹ et R² peuvent former, avec le carbone auquel ils sont liés sur le cycle, un carbocycle ou un hétérocycle ayant de 5 à 7 atomes, ces cycles pouvant être fusionnés avec un phényl, éventuellement substitué par 1 à 3 groupes choisis parmi R⁶ ;
- R³ représente :
   - un hydrogène ou un radical C₁-C₂ alkyle éventuellement halogéné, lorsque p égale 0 ou (M)p est un radical CH₂,
   - un radical C₁-C₂ alkyle éventuellement halogéné, lorsque (M)p représente un atome d'oxygène ou de soufre ;
- R⁴ représente :
   - l'atome d'hydrogène, ou
   - un groupe alkyl de 1 à 6 atomes de carbone, ou
   - un radical aryl, comprenant phényl, naphtyl, thiényl, furyl, pyridyl, pyrimidyl, pyridazinyl, pyrazinyl, benzothiényl, benzofuryl, quinolinyl, isoquinolinyl, ou méthylène dioxyphényl, éventuellement substitué par 1 à 3 groupements choisis parmi R⁶;
- R⁵ représente :
   - H, sauf quand R⁴ est H, ou
   - un radical alkyl de 1 à 6 atomes de carbone ou
   - acyl, de 2 à 6 atomes de carbone ou
   - un radical phényl; éventuellement substitué par 1 à 3 groupes parmi R⁶;
- R⁶ représente :
   - un atome d'halogène ou
   - un radical alkyl, haloalkyl, alkoxy, haloalkoxy, alkylthio, haloalkylthio ou alkylsulfonyl de 1 à 6 atomes de carbone ou
   - un radical cycloalkyl, halocycloalkyl, alcényloxy, alcynyloxy, alcénylthio, alcynylthio de 3 à 6 atomes de carbone ou
   - le groupe nitro ou cyano ou
   - un radical amino éventuellement mono ou disubstitué par un radical alkyl ou acyl de 1 à 6 atomes de carbone ou alkoxycarbonyl de 2 à 6 atomes de carbone
   - un radical phényl, phénoxy ou pyridyloxy, ces radicaux étant éventuellement substitués par 1 à 3 groupements, identiques ou différents, choisis parmi R⁷,
- R⁷ représente :
   - un atome d'halogène choisi parmi le fluor, le chlore,le brome, l'iode ou
   - un radical alkyle, linéaire ou ramifié, contenant de 1 à 6 atomes de carbone, ou
   - un radical alkoxy ou alkylthio, linéaire ou ramifié, contenant de 1 à 6 atomes de carbone ou
   - un radical haloalkoxy ou haloalkylthio, linéaire ou ramifié, contenant de 1 à 6 atomes de carbone ou
   - un radical nitrile ou
   - un radical nitro.

L'invention concerne également les formes salifiées acceptables en agriculture des composés ci-dessus définis.

Selon une variante préférée de l'invention, les composés optiquement actifs selon l'invention ont pour formule II : 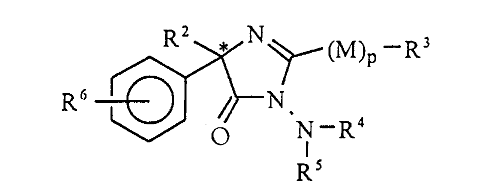 dans laquelle les différents symboles ont la même signification que dans la formule I.

Le mode de préparation des composés de formule I est indiqué dans les paragraphes qui suivent, selon deux variantes de procédés A et B. Les symboles représentés sur la formule I, qui apparaissent dans cette description du mode de préparation, conservent la même signification que dans la définition générale de l'invention, à moins qu' une autre définition ne leur soit expressément attribuée.

Les exemples ci-après illustrent les dérivés optiquement actifs de formule I et leur procédé de préparation.

Les structures de tous les dérivés illustrés ont été caractérisées à l'aide d'au moins une des techniques spectrales suivantes : sptectrométrie RMN du proton, spectrométrie RMN du carbone 13, spectrométrie Infra-Rouge et spectrométrie de masse, ainsi que les méthodes usuelles de mesure des pouvoirs rotatoires. Les excès énantiomèriques ont été déterminés soit par chromatographie liquide haute performance sur phase chirale, soit par RMN.

Dans les tableaux ci-dessous, les radicaux phényle, méthyle, éthyle, sont respectivement représentés par Ph, Me, Et.

### Variante A :

### Première étape :

On décrit dans une première étape de cette variante la préparation des isomères optiques de formule I à partir d'α aminoacides optiquement purs ou fortement enrichis en un énantiomère. On entend par composé optiquement actif fortement enrichi en un énantiomère déterminé, un composé contenant au moins 80%, de préférence 95% de cet énantiomère.

Les isomères optiques de formule I sont préparés selon 3 séries de procédés, selon la signification du groupe (M)ₚ-R³.

### 1°) Préparation des composés de formule I dans laquelle p = 1 et M = S :

Les composés de formule I dans laquelle p = 1 et M = S sont préparés par réaction du composé de formule III : 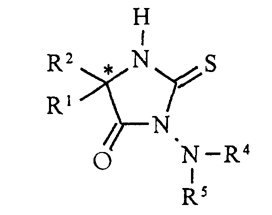 avec le composé de formule R³X, dans laquelle X représente l'atome de chlore, de brome ou d'iode ou le groupe sulfate, ou un groupe alkylsulfonyloxy ou arylsulfonyloxy, alkyl et aryl étant comme définis plus haut pour R¹ et R². La réaction s'effectue dans un solvant et en présence d'une base. Comme base on peut utiliser un alcoolate, par exemple du tertiobutylate de potassium, un hydroxyde alcalin ou alcalino-terreux, un carbonate alcalin ou une amine tertiaire. Comme solvant on peut utiliser les éthers, les éthers cycliques, les esters d'alkyls, l'acétonitrile, les alcools de 1 à 3 atomes de carbone, les solvants aromatiques, par exemple le tétrahydrofurane à température comprise entre -5°C et +80°C.
Une variante de la méthode décrite précédemment consiste à utiliser le procédé dit " one-pot" (schéma 1 ) comme décrit dans la demande de brevet européen EP 551048. Cette méthode consiste à partir directement de l'isothiocyanate de formule IV qui est traité par un composé de formule V dans un solvant et en présence d'une base comme décrit précédemment. L'intermédiaire de formule IIIa sous forme de sel n'est pas isolé mais traité directement avec le composé de formule R₃X dans lequel X a la même signification que précédemment.

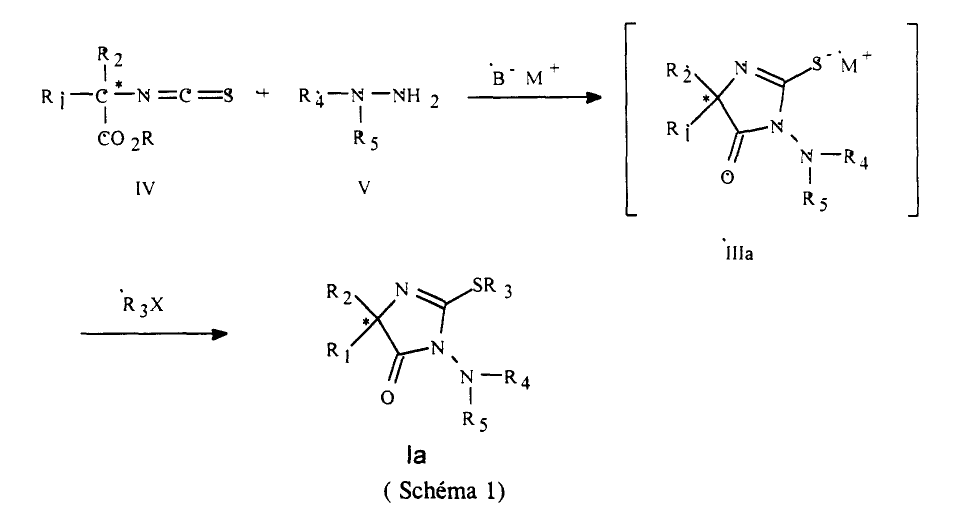

### Exemple 1: (+) (4-S) 4-méthyl-2-méthylthio-4-phényl-1-phénylamino-2-imidazoline-5-one (composé n°1)

Dans un réacteur de 20 l parcouru par un courant d'argon, on introduit 682 g (3,08 moles) de (+) (2-S) 2-phényl-2-isothiocyanato propionate de méthyle dissous dans 4 1 de tétrahydrofurane anhydre. On refroidit à 15°C. On coule 343 g (3,08 moles) de phénylhydrazine dissoute dans 2 l de tétrahydrofurane en 30 mn en maintenant la température entre 15°C et 18°C. Le milieu est maintenu sous agitation pendant 40 mn puis refroidit à 0°. On coule une solution de 346 g (3,08 moles) de tertiobutylate de potassium dans 4 l de tétrahydrofurane en 1 heure tout en maintenant la température à 0°C. L'agitation du milieu est pousuivie pendant 2 heures à 0°C et on observe la formation d'un précipité rose clair. On coule 218 ml (3,39 moles) d'iodure de méthyle en 15 mn en maintenant la température entre 0°C et 3°C puis on laisse remonter la température à l'ambiante en maintenant l'agitation pendant 2 heures. Le mélange réactionnel est versé sur 5 l d'eau. Après décantation, la phase aqueuse est extraite avec 3 fois 3 l d'acétate d'éthyle. Les phases organiques rassemblées sont lavées avec 5 l d'eau, séchées sur sulfate de magnésium puis concentrées sous pression réduite. On obtient 1099 g d'un solide brun. Celui-ci est recristallisé dans 2 l de toluène.
On obtient, après séchage, 555 g de (+) (4-S) 4-méthyl-2-méthylthio-4-phényl-1-phénylamino-2-imidazoline-5-one sous la forme d'un solide blanc cassé fondant à 138°C(Rendement = 58 %;[α]_{D}^{27°C} = + 61,1° ( + ou - 2, 9° )(c = 0,86 dans l'éthanol); taux d'excès enantiomérique(e.e) > 98 %).

De la même manière les composés analogues de formule IIa suivants ont été obtenus:

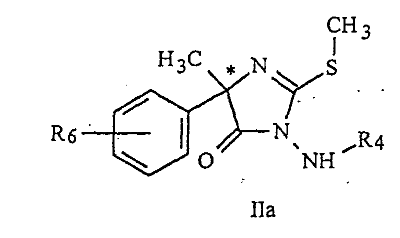

| *N° composé* | *R*^{*4*} | *R*^{*6*} | *(*α*)*_{*D*} *(C) Solvant* | *pF(°C)* | *Rdt(%)* |
|---|---|---|---|---|---|
| *1* | *Ph* | *H* | +*61° (0,8) EtOH* | *138* | *58* |

Le composé de formule III, peut être préparé par une réaction de cyclisation, entre un isothiocyanate de formule IV: 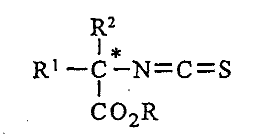 dans laquelle R représente un C₁-C₄ alkyl, et un composé de formule V:

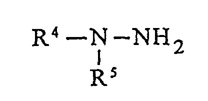

La réaction de cyclisation peut être réalisée de deux façons :
- thermiquement : dans ce cas on chauffe le mélange des réactifs à température comprise entre 110°C et 180°C dans un solvant aromatique tel que le toluène, le xylène, les chlorobenzènes.
- en milieu basique : on procède en présence d'un équivalent d'une base telle qu'un alcoolate alcalin, un hydroxyde alcalin ou une amine tertiaire. Dans ces conditions la cyclisation se produit à température comprise entre -10 et +80°C. Comme solvant on peut utiliser, notamment, les éthers, les éthers cycliques, les alcools, les esters, le DMF, le DMSO.

### Exemple 2 : (+) (4-S) 4-méthyl-4-phényl-1-phénylammo-2-thiohydantoïne (composé n°7).

Dans un tricol de 100 ml sous atmosphère d'azote sec, on introduit 0,7 g (0,00316 mole) de (+) (2-S) 2-isothiocyanato-2-phényl propionate de méthyle dilué dans 15 ml de tétrahydrofurane sec. On coule à 20°C en une seule fois 0,32 ml (0,00316 mole) de phénylhydrazine diluée dans 5 ml de tétrahydrofurane. La température s'élève de 2°C. Le milieu est maintenu 30 mn sous agitation magnétique. Apparition d'un précipité beige foncé. Le milieu est neutralisé par 0,4 ml d'acide acétique puis traité avec 20 ml d'eau. Après décantation, la phase aqueuse est extraite par 3 fois 20 ml d'éther éthylique. Les phases organiques sont rassemblées, lavées avec 2 fois 30 ml d'eau, séchées sur sulfate de magnésium puis concentrées sous pression réduite. Le résidu solide obtenu est chromatographié sur colone de silice en utilisant un mélange éluant composé d'heptane et d'acétate d'éthyle dans les proportions 50/50.

On recueille 0,55 g de (+) (4-S) 4-méthyl-4-phényl-1-phénylamino-2-thiohydantoïne sous la forme d'un solide beige fondant à 167°C(Rendement = 58 %;[α]_{D}^{27°C} = + 86° ( + ou - 3,2° ) ( c = 0,8 dans le méthanol ).

Les isothiocyanates de formule IV peuvent être préparés selon un des procédés cités dans Sulfur Reports Volume 8 (5) pages 327-375 (1989), à partir de l'α-aminoacide de formule VI via l'amino ester de formule X: 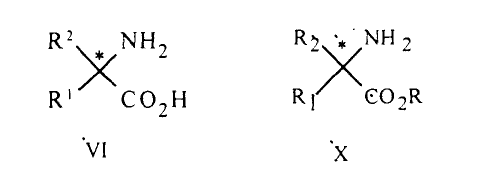 d'une manière bien connue de l'homme du métier.

### Exemple 3 : (+) (2-S) 2-isothiocyanato-2-phényl propionate de méthyle (composé n°8)

Dans un réacteur de 20 l, on introduit 780 g (3,61 moles) de (+) chlorydrate de (2-S) 2-amino-2-phényl propionate de méthyle puis 3,4 l d'eau. La température est ammenée à 20 °C. On ajoute 3,4 l de toluène puis on additionne par fraction 911 g (10,8 moles) d'hydrogénocarbonate de sodium pendant 1 heure. La température descent à 8-9°C. On coule 276 ml (3,61 moles) de thiophosgène pendant 2 heures. La réaction s'accompagne d'un dégagement gazeux et d'une élévation de température qui atteind 24°C en fin de coulée. Le milieu est encore maintenu 2 heures sous agitation. Après décantation, la phase aqueuse est extraite avec 2 l de toluène. Les phases toluèniques réunies sont lavées avec 4 l d'eau puis séchées sur sulfate de magnésium. La solution est concentrée sous pression réduite.

On obtient 682 g de (+) (2-S) 2-isothiocyanato-2-phényl propionate de méthyle sous la forme d'une huile légèrement colorée( .Rendement = 85 %;[α ]_{D}^{29°C} = + 16° ( + ou - 6,4° )( c = 0,78 dans le chloroforme )
De la même manière les composés analogues de formule IVa suivants ont été obtenus:

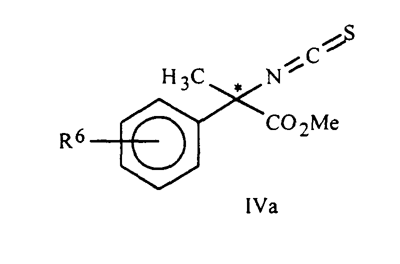

| *N° composé* | *R*^{*6*} | *(*α*)*_{*D*} *C Solvant* | *état physique* | *Rdt (%)* |
|---|---|---|---|---|
| *8* | *H* | +*16° (0,78) CHCl*_{*3*} | *Huile* | *85* |
| *17* | *4-F* | *(*+*)* | *Huile* | *72* |
| *18* | *4-F* | *(-)* | *Huile* | *80* |
| *19* | *4-(4-FPh)O* | *(*+*)* | *Huile* | *61* |
| *20* | *4-(4-FPh)O* | *-11° (0,7) EtOH* | *Huile* | *70* |

Les aminoesters de structure X peuvent être obtenus de manière connue soit par :
- amination diastéréosélective d'un composé prochiral suivie d'une déprotection de la copule chirale comme décrit par R.S.ATKINSON et coll., Tetrahedron, 1992, 48, pp 7713-30.
- dédoublement du racémique correspondant avec un composé chiral comme décrit par Y.SUGI et S.MITSUI, Bull. Chem. Soc. Japan, 1969, 42, pp 2984-89.
- estérification d'un aminoacide chiral comme décrit par D.J.CRAM et coll., J.Am.Chem.Soc., 1961, 83, pp 2183-89.

### Exemple 4 : (+) chlorydrate de (2-S) 2-amino-2-phényl propionate de méthyle (composé n° 9).

Dans un réacteur de 10 l, on charge 611 g (3,7 moles) d'acide (+) 2-amino-2-phényl propionique auquel on ajoute 5 1 de méthanol. On coule sur la suspension blanche formée 819 ml (11,22 moles) de chlorure de thionyle pendant 2 heures. La température atteind 58°C en fin de coulée. On observe un dégagement gazeux important qui est piégé par une solution de soude diluée. Le milieu est chauffé à 65°C pendant 14 heures. La solution est ensuite concentrée sous pression réduite. Le solide obtenu est traité par 1 l de toluène, filtré puis séché sous vide.

On obtient 762 g de (+) chlorydrate de (2-S) 2-amino-2-phényl propionate de méthyle sous la forme d'une poudre blanche fondant à 162°C(.Rendement = 62 %; [α]_{D}^{29°C} = + 53,3° ( + ou - 3,3° ) c = 0,75 dans l'eau ).
De la même manière les composés analogues de formule Xa suivants ont été obtenus:

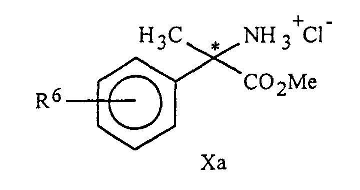

| *N°composé* | *R*^{*6*} | *(α)*_{*D*} *C Solvant* | *état physique* | *pF (°C)* | *Rdt (%)* |
|---|---|---|---|---|---|
| *9* | *H* | +*54° (0,91) CHCl*_{*3*} | *cristaux blancs* | *162* | *62* |
| *21* | *4-F* | *-61° (0,9) EtOH* | *solide blanc* | *50-60°C* | *93* |
| *22* | *4-F* | *(-)* | *solide blanc* | *-* | *95* |
| *23* | *4-(4-FPh)O* | *(*+*)* | *solide blanc* | *-* | *87* |
| *24* | *4-(4-FPh)O* | *(-)* | *solide blanc* | *-* | *95* |

Le (+) (2-S) 2-amino-2-phényl propionate de méthyle est obtenu par traitement du chlorydrate précedemment préparé avec 1 équivalent d'hydrogénocarbonate de sodium puis extraction au dichlorométhane. Il se présente sous la forme d'une huile incolore légèrement visqueuse( [α ]_{D}^{29°C} = + 54,8° ( + ou - 2,7° ) ( c = 0,91 dans le chloroforme ) e.e > 95 %).

### 2°) Préparation des isomères optiques de formule I dans laquelle p = 1 et M = O :

Les composés de formule I dans laquelle p = 1 et M = O , sont préparés en faisant réagir le composé de formule I correspondant pour lequel p = 1 et M = S selon un procédé décrit dans la demande de brevet européen EP 599749, avec l'alcool de formule R³OH, dans un solvant, en présence d'une base forte, et à une température comprise entre 50 et 80 °C. Comme base forte, on peut utiliser un alcoolate alcalin R³O⁻Met. ⁺, dans lequel Met. ⁺ représente un métal alcalin ou alcalinoterreux, un hydroxyde alcalin ou une base organique forte. La réaction est effectuée de préférence en prenant pour solvant l'alccol R³OH et en utilisant l'alcoolate de sodium correspondant R³O⁻Na⁺ comme base.

### Exemple 5: (+) (4-S) 4-méthyl-2-méthoxy-4-phényl-1-phénylamino-2-imidazoline-5-one(composé n°3).

Dans un ballon tricol de 250 ml sous atmosphère d'azote sec, on introduit 80 ml de méthanol puis 0,74 g (0,032 mole) de sodium coupé en morceaux fins. On ajoute ensuite 5 g (0,016 mole) de (+) (4-S) 4-méthyl-2-méthylthio-4-phényl-1-phénylamino-2-imidazoline-5-one. On porte à reflux pendant 20h. On refroidit à température ambiante puis on acidifie avec 0,5 ml d'acide acétique. Le méthanol est éliminé par distillation sous pression réduite puis le résidu obtenu est repris par 50 ml d'éther éthylique, lavé avec 3 fois 40 ml d'eau, séché sur sulfate de magnésium puis la solution est concentrée sous pression réduite. On obtient un miel rougeâtre qui est purifié par chromatographie sur colone de silice avec pour éluant un mélange heptane/acétate d'éthyle 70/30.

On obtient 2 g de (+) (4-S) 4-méthyl-2-méthoxy-4-phényl-1-phénylamino-2-imidazoline-5-one sous la forme d'une poudre rose clair fondant à 132°C(Rendement = 42%; [α]_{D}^{25°C} = + 53,1° ( + ou - 2,4° ) ( c = 1 dans le méthanol ); e.e > 98%.

De la même manière les composés analogues de formule Ib suivants ont été obtenus:

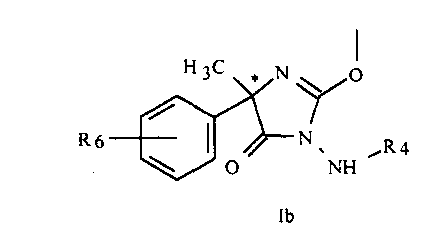

| *N°composé* | *R*^{*4*} | *R*^{*6*} | *(α)*_{*D*} *C Solvant* | *pF(°C)* | *Rdt(%)* |
|---|---|---|---|---|---|
| *3* | *Ph* | *H* | +*53° (1,0) MeOH* | *132* | *42* |

### 3°) Préparation des isomères optiques de formule I dans laquelle p = 0 :

Les composés de formule I dans laquelle p = 0 et R³ est un atome d'hydrogène sont obtenus à partir du composé de formule VII : 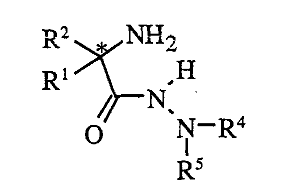 en faisant réagir celui-ci avec le diméthylacétal du diméthylformamide (DMFDMA). La réaction est effectuée à une température comprise entre 10 et 100° C, dans le DMFDMA en excès.

Le composé de formule VII est préparé à partir d'un composé de formule VIII : 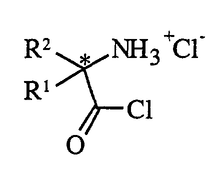 par réaction de ce dernier avec le composé de formule V, à une température comprise entre -20 et 40 °C, dans un solvant constitué d'un éther cyclique ou non cyclique, éventuellement en présence d'une base. La base est
par réaction de ce dernier avec le composé de formule V, à une température comprise entre -20 et 40 °C, dans un solvant constitué d'un éther cyclique ou non cyclique, éventuellement en présence d'une base. La base est choisie parmi les bases organiques azotées telle que la triéthylamine ou la pyridine.

Les composés de formule VIII peuvent être obtenus, à partir de l'α-aminoacide de formule VI en considérant la méthode décrite par S. Levine dans J. Am. Chem. Soc. de 1953, volume 76, page 1392.

Les composés optiquement actifs de formule I, dans laquelle R³ est un radical C₁-C₂ alkyl, éventuellement halogéné, et dans laquelle p = 0 ou p = 1 et M = CH₂ sont obtenus à partir du composé de formule IX : 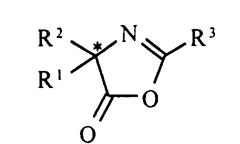 dans laquelle R³ représente un radical C₁-C₃ alkyl,
par réaction de celui-ci avec le composé de formule V, dans des conditions déduites, par analogie, de la méthode exposée dans l'article de J. P. Branquet et al. dans Bull. Soc. Chim. de France, 1965, (10), pp 2942-2954.

Ce même article donne un mode opératoire au terme duquel le composé de formule IX peut être préparé à partir de l'a-aminoacide de formule VI.

### Deuxième étape :

On précise dans cette deuxième étape le mode d'accès aux α-aminoacides optiquement purs ou fortement enrichis, de formule VI utilisés dans l'étape précédente.

Ces α-aminoacides peuvent être obtenus selon l'une des méthodes suivantes :
- soit par synthèse diastéréosélective puis suppression de la copule chirale, tel que décrit par M. Chaari, A. Jenhi, J. P. Lavergne et P. Viallefont dans Tetrahedron 1991 Tome 4 pages 4619-4630.
- soit par résolution enzymatique de l'amide racémique, méthode pour laquelle on pourra utilement consulter les références suivantes :
   R. M. Kellog, E. M. Meijer et coll. J. Org. Chem. 1988 Tome 53, pages 1826-1828
   D. Rossi et A. Calcagni Experimentia 1985 volume 41 pages 35-37.
- soit par hydrolyse d'un précurseur chiral d'amino acide tel par exemple:
   - un formyl aminoacide de structure XI tel que décrit par Mac KENZIE et CLOUGH, J. Chem. Soc., 1912, pp 390-397 ou par D.J.CRAM et coll., J. Am. Chem. Soc., 1961, 83, pp 2183-89.
   - une hydantoïne de structure XII tel que décrit dans la demande de brevet britannique publiée n°1201169.

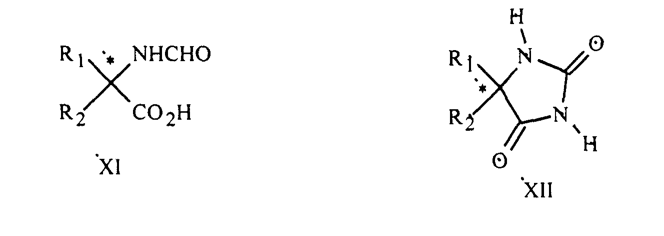

Les composés de formules XI ou XII peuvent être obtenus par dédoublement du mélange racémique correspondant avec un composé chiral comme décrit par Mac KENZIE et CLOUGH, J. Chem. Soc., 1912, pp 390-397 ou par D.J.CRAM et coll., J. Am. Chem. Soc., 1961, 83, pp 2183-89 pour le composé XI ou comme décrit dans la demande de brevet international n° 9208702 publiée pour le composé XII.

### Exemple 6 : acide (+) (2-S) 2-amino-2-phényl propionique ( composé n°10).

Dans un autoclave de 1 litre, on introduit successivement 22 g (0,115 mole) de (+) (5-S) 5-méthyl-5-phényl hydantoïne, 100 ml d'eau et 100 ml d'ammoniaque à 28 %. Le milieu est chauffé à 160°C pendant 15 heures. Après refroidissement à température ambiante, la solution est concentrée sous pression réduite. Le solide blanc obtenu est traité avec 100 ml d'acétate d'éthyle pendant 2 heures puis filtré et séché sous vide à 80°C.
On recueille 10,5 g d'acide (+) (2-S) 2-amino-2-phényl propionique sous la forme d'une poudre blanche qui présente une température de décomposition de 266°C (Rendement = 55 %; [α ]_{D}^{27°C} = + 71,9° ( + ou - 3,1° )( c = 0,8 dans l'acide chlorydrique 1 N).

De la même manière les composés analogues de formule VIa suivants ont été obtenus:

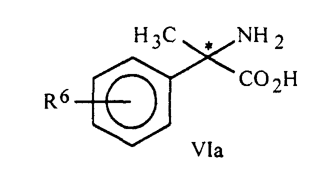

| *N°composé* | *R*^{*6*} | *(α)*_{*D*} *C Solvant* | *pF (°C)* | *Rdt (%)* |
|---|---|---|---|---|
| *10* | *H* | +*72*°*(0,8) HCl 1N* | *266°C* | *55* |
| *31* | *4-F* | *(*+*)* | *-* | *44* |
| *32* | *4-F* | *(-)* | *-* | *92* |
| *33* | *4-(4-FPh)O* | *(*+*)* | *-* | *87* |
| *34* | *4-(4-FPh)O* | *(-)* | *-* | *76* |

L'exemple 9 illustre la préparation des composés de formule XII

### Exemple 9 : 5-S(+) 5-méthyl-5-phényl hydantoïne (composé n° 35).

A une suspention agitée de 70,0 gr ( 0,368 mol) de (5-R,S) 5-méthyl-5-phényl hydantoïne dans 2000 ml d'eau est ajouté 5,6 gr (0,139 mol) d'hydroxyde de sodium . La solution obtenue est portée à 40°C puis on ajoute 44,6 gr (0,368 mol) de R-(+)a-méthylbenzylamine. La solution obtenue est maitenue à 50°C pendant 0,75h, un précipité blanc apparait au bout de 3mn . En fin de chauffage on laisse cristalliser le milieu réactionnel pendant 24h puis les cristaux son filtrés, lavés avec 70ml d'eau , essorés sous courant d'air pendant 2h, on récupère 45gr d'un solide blanc que l'on ajoute à 220 ml d'acide chlorydrique 1N à 10°C. La suspention obtenue est agitée pendant 2h puis les cristaux son filtrés, lavés avec 100ml d'eau , essorés puis séchés sous pression réduite à 50°C pendant 15h. On récupère ainsi 23gr (0,121 mol) de 5-S(+) 5-méthyl-5-phényl hydantoïne sous la forme d'un solide blanc cassé fondant à 242°C(Rendement = 66%; [α]_{D}^{29°C} = + 113° (c = 1,O dans l'éthanol);
De la même manière mais en utilisant de la S-(-)α-méthylbenzylamine On récupère de 5-R(-) 5-méthyl-5-phényl hydantoïne sous la forme d'un solide blanc cassé fondant à 248°C(Rendement = 54%;[α ]_{D}^{29°C} = + 120° (c = 1,O dans l'éthanol);
De la même manière les composés analogues de formule XIIa suivants ont été obtenus:

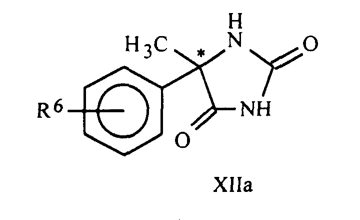

| *N°composé* | *R*^{*6*} | *(α)*_{*D*} *C solvant* | *pF (°C)* | *Rdt (%)* |
|---|---|---|---|---|
| *35* | *H* | + *113° (1,O) EtOH* | *242* | *66* |
| *36* | *H* | *-120° (1,0) EtOH* | *248* | *54* |
| *37* | *4-F* | +*111° (0,8) EtOH* | *230* | *44* |
| *38* | *4-F* | *-114° (0,8) EtOH* | *230* | *31* |
| *39* | *4-(4-FPh)O* | +*54° (0,5) EtOH* | *190* | *-* |
| *40* | *4-(4-FPh)O* | *-57° (0,6) EtOH* | *189* | *40* |

### Variante B :

Selon une deuxième variante du procédé de préparation des isomères optiques de formule I, ceux-ci sont obtenus à partir des composés racémiques correspondants, par chromatographie liquide haute performance sur phase stationnaire chirale. Une phase stationnaire chirale de type PIRKLE à greffon D phényl glycine est préférée.

Les composés racémiques correspondant à la formule I sont préparés selon les méthodes décrites dans les trois demandes de brevet citées dans la partie introductive du présent texte.

Les exemples ci-après illustrent les dérivés optiquement actifs de formule I obtenus selon la variante B du procédé de préparation.

### Exemple 7 :Séparation des énantiomères (+) et (-) du composé de formule suivante (composés n° 1 et 2) ;

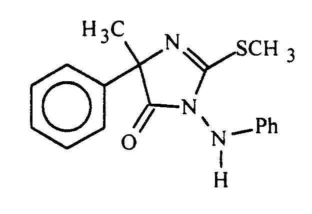

Le composé racémique correspondant est préparé selon un mode opératoire analogue à celui décrit dans l'exemple 1 de la demande de brevet EP 551048 déjà citée. Ce composé racémique est dissous dans un mélange éluant composé de n heptane, isopropanol et dichlorométhane, dans la proportion pondérale respective de 93, 5, et 2 %.

On injecte 2,3 ml du mélange ainsi obtenu dans la colonne chirale chromatographique haute performance, de caractéristiques suivantes :
- colonne de type PIRKLE, de diamètre 10 mm et de longueur 250 mm;
- support : silice 5 *µ*m 100 angström à greffage D phényl glycine ionique.

Le débit choisi est de 10 ml/mn et le détecteur utilisé est un détecteur UV à 250 nm. Les composés énantiomériquement purs sont récupérés par fractionnement et concentration des fractions pures.

On a rassemblé, dans le tableau ci-dessous, les caractéristiques physiques des énantiomères obtenus, à savoir le point de fusion PF, le pouvoir rotatoire [α]_{D}²⁰ mesuré en degrés pour le composé dissous dans l'éthanol à la concentration de 0,5 g pour 100 ml, et le temps de rétention t_{R} :

| **Composé n°** | **PF (°C)** | **[α**]_{D}²⁰ | t_{R} (en minutes) |
|---|---|---|---|
| 1 | 138 | + 60,7 + ou - 1,3 | 5,73 |
| 2 | 138 | -59,6+ ou - 0,9 | 6,55 |

### Exemple 8 : Séparation des énantiomères (+) et (-) du composé de formule suivante ( composés n° 3 et 4 ):

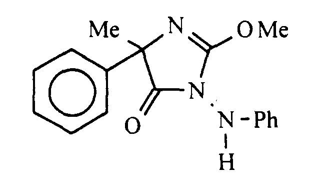

Le composé racémique correspondant est préparé selon un mode opératoire analogue à celui décrit dans l'exemple 1 de la demande de brevet EP 599749 déjà citée. Les énantiomères (+) et (-) correspondants (respectivement composés n° 3 et 4) sont obtenus en procédant de la même manière que précédemment. Le volume injecté dans la colonne chirale est de 1,5 ml. Le pouvoir rotatoire est mesuré après dissolution des composés dans le méthanol et figure avec les autres caractéristiques physiques, identiques à celles déterminées précédemment, dans le tableau ci-dessous :

| **Composé n°** | **PF (°C)** | **[α]**_{**D**}^{**20**} | **t**_{**R**} **(en minutes)** |
|---|---|---|---|
| 3 | 132 | + 51,3 + ou - 1,2 | 9,89 |
| 4 | 132 | - 53,2 + ou - 1,3 | 11,17 |

La configuration absolue des composés N° 1 à 4 a été déterminée par corrélation chimique avec la configuration absolue de l'α-aminoacide correspondant décrite dans la littérature.

L'invention a également pour objet de nouveaux composés optiquement actifs utiles notamment comme intermédiaires dans la préparation des composés de formule I. Ces intermédiaires ont pour formules III, VII, : 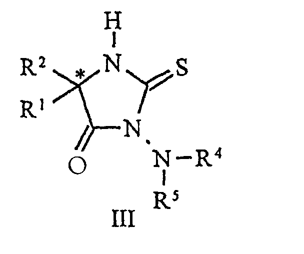 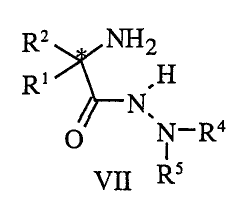 dans lesquelles R¹ à R⁵ ont les mêmes significations que dans la formule générale I de l'invention,
et le composé de formule IX : 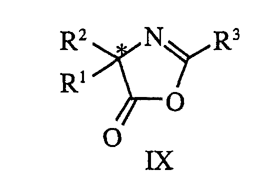 dans laquelle R¹ et R² ont la même signification que précédemment et R³ représente un radical C₁-C₃ alkyl éventuellement halogéné,
et le composé de formule XIIb: 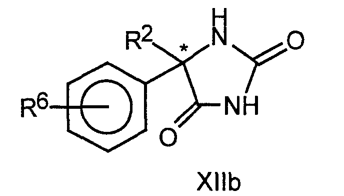 dans laquelle R¹ a la même signification que précédemment, R⁶ représente un radical phényl, phénoxy ou pyridyloxy, ces radicaux étant éventuellement substitués par 1 à 3 groupements, identiques ou différents, choisis parmi R⁷ tel que défini précédemment .

Les exemples suivants illustrent les propriétés fongicides des composés de formule (I) selon l'invention n°1 à 4. Dans ces exemples, le mélange racémique correspondant aux composés énantiomères 1 et 2 est noté 1+2. De même le mélange racémique correspondant aux composés 3 et 4 est noté 3+4. Plus généralement, le mélange racémique correspondant aux composés énantiomères n et n+1 est noté n+(n+1).

### Exemple B1 : Test in vivo sur Puccinia recondita (rouille du blé) :

On prépare, par broyage fin, une suspension aqueuse de la matière active à tester ayant la composition suivante:
- matière active: 60 mg
- agent tensioactif Tween 80, (oléate de dérivé polyoxyéthyléné du sorbitan) dilué à 10% dans l'eau : 0,3 ml
- on complète à 60 ml d'eau.

La matière active à tester est soit l'un des 2 énantiomères selon l'invention, soit le mélange racémique correspondant.

Cette suspension aqueuse est ensuite diluée par de l'eau pour obtenir la concentration désirée en matière active.

Du blé de la variété Talent, en godets, semé sur un substrat tourbe terre pouzzolane 50/50, est traité au stade 10 cm de hauteur par pulvérisation de la suspension aqueuse ci dessus.

Au bout de 24 heures, une suspension aqueuse de spores (100000 sp/cm³) est pulvérisée sur le blé; cette suspension a été obtenue à partir de plants contaminés. On place ensuite le blé pendant 24 heures en cellule d'incubation à environ 20°C et à 100% d'humidité relative, puis pendant 7 à 14 jours à 60% d'humidité relative.

Le contrôle de l'état des plants se fait entre le 8ème et le 15ème jour après la contamination, par comparaison avec un témoin non traité. On détermine ensuite la concentration en matière active testée, CI₇₅, (exprimée en ppm) pour laquelle on observe une inhibition de 75% de la maladie.

Les résultats sont rassemblés dans le tableau suivant :

| ***N° composé*** | ***CI75 (ppm)*** |
|---|---|
| ***1+2*** | ***330*** |
| ***1*** | ***37*** |
| ***2*** | ***>1000*** |
| ***3+4*** | ***330*** |
| ***3*** | ***110*** |
| ***4*** | ***> 1000*** |

### Exemple B2 : Test in vivo sur Phytophthora infestans (mildiou de la tomate) :

On prépare, par broyage fin, une suspension aqueuse de la matière active à tester ayant la composition suivante:
- matière active: 60 mg
- agent tensioactif Tween 80, (oléate de dérivé polyoxyéthyléné du sorbitan) dilué à 10% dans l'eau : 0,3 ml
- on complète à 60 ml d'eau.

La matière active à tester est choisie parmi les mêmes composés que dans l'exemple précédent.

Cette suspension aqueuse est ensuite diluée par de l'eau pour obtenir la concentration désirée en matière active.

Des plants de tomate (variété Marmande) sont cultivés dans des godets. Lorsque ces plants sont agés d'un mois (stade 5 à 6 feuilles, hauteur 12 à 15 cm), ils sont traités par pulvérisation de la suspension aqueuse ci dessus et à diverses concentrations du composé à tester.

Au bout de 24 heures, on contamine chaque plant par pulvérisation au moyen d'une suspension aqueuse de spores (30000 sp/cm³) de Phytophthora infestans.

Après cette contamination, les plants de tomate sont mis en incubation pendant 7 jours à 20°C environ en atmosphère saturée d'humidité.

Sept jours après la contamination, on compare les résultats obtenus dans le cas des plants traités par la matière active à tester à ceux obtenus dans le cas des plants utilisés comme témoins. On détermine ensuite la concentration en matière active testée, CI₇₅, (exprimée en ppm) pour laquelle on observe une inhibition de 75% de la maladie.

Les résultats sont rassemblés dans le tableau suivant :

| ***N° composé*** | ***CI75 (ppm)*** |
|---|---|
| *1+2* | *110* |
| *1* | *37* |
| *2* | *>1000* |
| *3+4* | *330* |
| *3* | *110* |
| *4* | *>1000* |

L'invention concerne également les compositions pour la protection des plantes contre les maladies fongiques, comprenant, en association avec un ou des supports solides ou liquides acceptables en agriculture et/ou des agens tensio-actifs également acceptables en agriculture, une (ou plusieurs) matière active qui est un composé de formule I.

En effet, pour leur emploi pratique, les composés selon l'invention sont rarement utilisés seuls. Le plus souvent ces composés font partie de compositions. Ces compositions, utilisables comme agents fongicides, contiennent comme matière active un composé selon l'invention tel que décrit précédemment en mélange avec les supports solides ou liquides, acceptables en agriculture et les agents tensio-actifs également acceptables en agriculture. En particulier sont utilisables les supports inertes et usuels et les agents tensio-actifs usuels. Ces compositions font également partie de l'invention.

Ces compositions peuvent contenir aussi toute sorte d'autres ingrédients tels que, par exemple, des colloïdes protecteurs, des adhésifs, des épaississants, des agents thixotropes, des agents de pénétration, des stabilisants, des séquestrants, etc... Plus généralement les composés utilisés dans l'invention peuvent être combinés à tous les additifs solides ou liquides correspondant aux techniques habituelles de la mise en formulation.

D'une façon générale, les compositions selon l'invention contiennent habituellement de 0,05 à 95 % environ (en poids) d'un composé selon l'invention (appelé par la suite matière active), un ou plusieurs supports solides ou liquides et, éventuellement, un ou plusieurs agents tensioactifs.

Par le terme "support", dans le présent exposé, on désigne une matière organique ou minérale, naturelle ou synthétique, avec laquelle le composé est combiné pour faciliter son application sur la plante, sur des graines ou sur le sol. Ce support est donc généralement inerte et il doit être acceptable en agriculture, notamment sur la plante traitée. Le support peut être solide (argiles, silicates naturels ou synthétiques, silice, résines, cires, engrais solides, etc...) ou liquide (eau, alcools, notamment le butanol etc...).

L'agent tensioactif peut être un agent émulsionnant, dispersant ou mouillant de type ionique ou non ionique ou un mélange de tels agents tensioactifs. On peut citer par exemple des sels d'acides polyacryliques, des sels d'acides lignosulfoniques, des sels d'acides phénolsulfoniques ou naphtalènesulfoniques, des polycondensats d'oxyde d'éthylène sur des alcools gras ou sur des acides gras ou sur des amines grasses, des phénols substitués (notamment des alkylphénols ou des arylphénols), des sels d'esters d'acides sulfosucciniques, des dérivés de la taurine (notamment des alkyltaurates), des esters phosphoriques d'alcools ou de phénols polyoxyéthylés, des esters d'acides gras et de polyols, les dérivés à fonction sulfates, sulfonates et phosphates des composés précédents. La présence d'au moins un agent tensioactif est généralement indispensable lorsque le composé et/ou le support inerte ne sont pas solubles dans l'eau et que l'agent vecteur de l'application est l'eau.

Ainsi donc, les compositions à usage agricole selon l'invention peuvent contenir les matières actives selon l'invention dans de très larges limites, allant de 0,05 % à 95 % (en poids). Leur teneur en agent tensio-actif est avantageusement comprise entre 5 % et 40 % en poids.

Ces compositions selon l'invention sont elles-mêmes sous des formes assez diverses, solides ou liquides.

Comme formes de compositions solides, on peut citer les poudres pour poudrage (à teneur en composé pouvant aller jusqu'à 100 %) et les granulés, notamment ceux obtenus par extrusion, par compactage, par imprégnation d'un support granulé, par granulation à partir d'une poudre (la teneur en composé dans ces granulés étant entre 0,5 et 80 % pour ces derniers cas), les comprimés ou tablettes effervescents.

Les composés de formule (I) peuvent encore être utilisés sous forme de poudres pour poudrage ; on peut aussi utiliser une composition comprenant 50 g de matière active et 950 g de talc ; on peut aussi utiliser une composition comprenant 20 g de matière active, 10 g de silice finement divisée et 970 g de talc ; on mélange et broie ces constituants et on applique le mélange par poudrage.

Comme formes de compositions liquides ou destinées à constituer des compositions liquides lors de l'application, on peut citer les solutions, en particulier les concentrés solubles dans l'eau, les concentrés émulsionnables, les émulsions, les suspensions concentrées, les aérosols, les poudres mouillables (ou poudre à pulvériser), les pâtes, les gels.

Les concentrés émulsionnables ou solubles comprennent le plus souvent 10 à 80 % de matière active, les émulsions ou solutions prêtes à l'application contenant, quant à elles, 0,001 à 20 % de matière active.

En plus du solvant, les concentrés émulsionnables peuvent contenir quand c'est nécessaire, 2 à 20 % d'additifs appropriés comme les stabilisants, les agents tensio-actifs, les agents de pénétration, les inhibiteurs de corrosion, les colorants ou les adhésifs précédemment cités.

A partir de ces concentrés, on peut obtenir par dilution avec de l'eau des émulsions de toute concentration désirée, qui conviennent particulièrement à l'application sur les cultures.

A titre d'exemple, voici la composition de quelques concentrés émulsionnables :

### Exemple CE 1 :

- matière active 400 g/l
- dodécylbenzène sulfonate alcalin 24 g/l
- nonylphénol oxyéthylé à 10 molécules d'oxyde d'éthylène 16 g/l
- cyclohexanone 200 g/l
- solvant aromatique q.s.p.1 litre

Selon une autre formule de concentré émulsionnable, on utilise :

### Exemple CE 2

- matière active 250 g
- huile végétale époxydée 25 g
- mélange de sulfonate d'alcoylaryle et d'éther de polyglycol et d'alcools gras 100 g
- diméthylformamide 50 g
- xylène 575 g

Les suspensions concentrées, également applicables en pulvérisation, sont préparées de manière à obtenir un produit fluide stable ne se déposant pas et elles contiennent habituellement de 10 à 75 % de matière active, de 0,5 à 15 % d'agents tensioactifs, de 0,1 à 10 % d'agents thixotropes, de 0 à 10 % d'additifs appropriés, comme des anti-mousses, des inhibiteurs de corrosion, des stabilisants, des agents de pénétration et des adhésifs et, comme support, de l'eau ou un liquide organique dans lequel la matière active est peu ou pas soluble : certaines matières solides organiques ou des sels minéraux peuvent être dissous dans le support pour aider à empêcher la sédimentation ou comme antigels pour l'eau.

A titre d'exemple, voici une composition de suspension concentrée :

### Exemple SC 1 :

- matière active 500 g
- phosphate de tristyrylphénol polyéthoxylé 50 g
- alkylphénol polyéthoxylé 50 g
- polycarboxylate de sodium 20 g
- éthylène glycol 50 g
- huile organopolysiloxanique (antimousse) 1 g
- polysaccharide 1,5 g
- eau 316,5 g

Les poudres mouillables (ou poudre à pulvériser) sont habituellement préparées de manière qu'elles contiennent 20 à 95 % de matière active, et elles contiennent habituellement, en plus du support solide, de 0 à 30 % d'un agent mouillant, de 3 à 20 % d'un agent dispersant, et, quand c'est nécessaire, de 0,1 à 10 % d'un ou plusieurs stabilisants et/ou autres additifs, comme des agents de pénétration, des adhésifs, ou des agents antimottants, colorants, etc...

Pour obtenir les poudres à pulvériser ou poudres mouillables, on mélange intimement les matières actives dans les mélangeurs appropriés avec les substances additionnelles et on broie avec des moulins ou autres broyeurs appropriés. On obtient par là des poudres à pulvériser dont la mouillabilité et la mise en suspension sont avantageuses ; on peut les mettre en suspension avec de l'eau à toute concentration désirée et ces suspensions sont utilisables trés avantageusement en particulier pour l'application sur les feuilles des végétaux.

A la place des poudres mouillables, on peut réaliser des pâtes. Les conditions et modalités de réalisation et d'utilisation de ces pâtes sont semblables à celles des poudres mouillables ou poudres à pulvériser.

A titre d'exemple, voici diverses compositions de poudres mouillables (ou poudres à pulvériser) :

### Exemple PM 1

- matière active 50%
- alcool gras éthoxylé (agent mouillant) 2,5%
- phényléthylphénol éthoxylé (agent dispersant) 5 %
- craie (support inerte) 42,5%

### Exemple PM 2 :

- matière active 10%
- alcool synthétique oxo de type ramifié, en C13 éthoxylé par 8 à 10 oxyde d'éthylène (agent mouillant) 0,75%
- lignosulfonate de calcium neutre (agent dispersant) 12%
- carbonate de calcium (charge inerte) q.s.p. 100%

### Exemple PM 3 :

Cette poudre mouillable contient les mêmes ingrédients que dans l'exemple précédent, dans les proportions ci-après :
- matière active 75%
- agent mouillant 1,50%
- agent dispersant 8%
- carbonate de calcium (charge inerte) q.s.p. 100%

### Exemple PM 4 :

- matière active 90%
- alcool gras éthoxylé (agent mouillant) 4%
- phényléthylphénol éthoxylé (agent dispersant) 6%

### Exemple PM 5 :

- matière active 50%
- mélange de tensio-actifs anioniques et non ioniques (agent mouillant) 2,5%
- lignosulfonate de sodium (agent dispersant) 5 %
- argile kaolinique (support inerte) 42,5%

Les dispersions et émulsions aqueuses, par exemple les compositions obtenues en diluant à l'aide d'eau une poudre mouillable ou un concentré émulsionnable selon l'invention, sont comprises dans le cadre général de la présente invention. Les émulsions peuvent être du type eau-dans-l'huile ou huile-dans-l'eau et elles peuvent avoir une consistance épaisse comme celle d'une "mayonnaise".

Les composés selon l'invention peuvent être formulés sous la forme de granulés dispersibles dans l'eau également compris dans le cadre de l'invention.

Ces granulés dispersibles, de densité apparente généralement comprise entre environ 0,3 et 0,6 ont une dimension de particules généralement comprise entre environ 150 et 2000 et de préférence entre 300 et 1500 microns.

La teneur en matière active de ces granulés est généralement comprise entre environ 1 % et 90 %, et de préférence entre 25 % et 90 %.

Le reste du granulé est essentiellement composé d'une charge solide et éventuellement d'adjuvants tensio-actifs conférant au granulé des propriétés de dispersibilité dans l'eau. Ces granulés peuvent être essentiellement de deux types distincts selon que la charge retenue est soluble ou non dans l'eau. Lorsque la charge est hydrosoluble, elle peut être minérale ou, de préférence, organique. On a obtenu d'excellents résultats avec l'urée. Dans le cas d'une charge insoluble, celle-ci est de préférence minérale, comme par exemple le kaolin ou la bentonite. Elle est alors avantageusement accompagnée d'agents tensio-actifs (à raison de 2 à 20 % en poids du granulé) dont plus de la moitié est, par exemple, constituée par au moins un agent dispersant, essentiellement anionique, tel qu'un polynaphtalène sulfonate alcalin ou alcalino terreux ou un lignosulfonate alcalin ou alcalino-terreux, le reste étant constitué par des mouillants non ioniques ou anioniques tel qu'un alcoyl naphtalène sulfonate alcalin ou alcalino-terreux.

Par ailleurs, bien que cela ne soit pas indispensable, on peut ajouter d'autres adjuvants tels que des agents anti-mousse.

Le granulé selon l'invention peut être préparé par mélange des ingrédients nécessaires puis granulation selon plusieurs techniques en soi connues (drageoir, lit fluide, atomiseur, extrusion, etc...). On termine généralement par un concassage suivi d'un tamisage à la dimension de particule choisie dans les limites mentionnées ci-dessus. On peut encore utilisé des granulés obtenus comme précédemment puis imprégnés avec une composition contenant la matière active.

De préférence, il est obtenu par extrusion, en opérant comme indiqué dans les exemples ci-après.

### Exemple GD1 : Granulés dispersibles

Dans un mélangeur, on mélange 90 % en poids de matière active et 10 % d'urée en perles. Le mélange est ensuite broyé dans un broyeur à broches. On obtient une poudre que l'on humidifie avec environ 8 % en poids d'eau. La poudre humide est extrudée dans une extrudeuse à rouleau perforé. On obtient un granulé qui est séché, puis concassé et tamisé, de façon à ne garder respectivement que les granulés d'une dimension comprise entre 150 et 2000 microns.

### Exemple GD2 : Granulés dispersibles

Dans un mélangeur, on mélange les constituants suivants :
- matière active 75 %
- agent mouillant (alkylnaphtalène sulfonate de sodium) 2%
- agent dispersant (polynaphtalène sulfonate de sodium) 8%
- charge inerte insoluble dans l'eau (kaolin) 15%

Ce mélange est granulé en lit fluide, en présence d'eau, puis séché, concassé et tamisé de manière à obtenir des granulés de dimension comprise entre 0,15 et 0,80 mm.

Ces granulés peuvent être utilisés seuls, en solution ou dispersion dans de l'eau de manière à obtenir la dose cherchée. Ils peuvent aussi être utilisés pour préparer des associations avec d'autres matières actives, notamment fongicides, ces dernières étant sous la forme de poudres mouillables, ou de granulés ou suspensions aqueuses.

En ce qui concerne les compositions adaptées au stockage et au transport, elles contiennent plus avantageusement de 0,5 à 95 % (en poids) de substance active.

L'invention a également pour objet un procédé de traitement des cultures atteintes ou susceptibles d'être atteintes par les maladies fongiques, caractérisé en ce que l'on applique, de façon préventive ou curative, une quantité efficace d'un composé optiquement actif de formule I.

Les composés de formule I sont appliqués avantageusement à des doses de 0,005 à 5 kg/ha, et plus spécifiquement de 0,01 à 1 kg/ha.

## Revendications

1. Dérivés optiquement actifs de 2-imidazoline-5-ones de formule générale I : 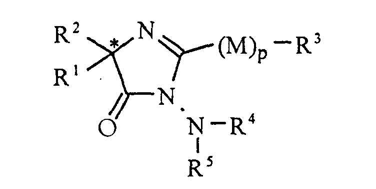 dans laquelle :
- M représente un atome d'oxygène ou de soufre, ou un radical CH₂, éventuellement halogéné ; p est un nombre entier égal à 0 ou 1 ;
- * signifie que l'atome de carbone asymétrique correspondant a une configuration stéréospécifique ;
- R¹ et R² sont différents et représentent :
- un radical alkyl ou haloalkyl de 1 à 6 atomes de carbone ou
- un radical alkoxyalkyl, alkylthioalkyl, alkylsulfonylalkyl, mono alkylaminoalkyl, alcènyl ou alcynyl de 2 à 6 atomes de carbone ou
- un radical dialkylaminoalkyl ou cycloalkyl de 3 à 7 atomes de carbone ou
- un radical aryl, comprenant phényl, naphtyl, thiényl, furyl, pyridyl, benzothiényl, benzofuryl, quinolinyl, isoquinolinyl, ou méthylène dioxyphényl, éventuellement substitué par 1 à 3 groupements choisis parmi R⁶ ou
- un radical arylalkyl, aryloxyalkyl , arylthioalkyl ou arylsulfonylalkyl, les termes aryl et alkyl ayant les définitions données ci-dessus ou
- R¹ et R² peuvent former, avec le carbone auquel ils sont liés sur le cycle, un carbocycle ou un hétérocycle ayant de 5 à 7 atomes, ces cycles pouvant être fusionnés avec un phényl, éventuellement substitué par 1 à 3 groupes choisis parmi R⁶ ;
- R³ représente :
- un hydrogène ou un radical C₁-C₂ alkyle éventuellement halogéné, lorsque p égale 0 ou (M)p est un radical CH₂,
- un radical C₁-C₂ alkyle éventuellement halogéné, lorsque (M)p représente un atome d'oxygène ou de soufre ;
- R⁴ représente :
- l'atome d'hydrogène, ou
- un groupe alkyl de 1 à 6 atomes de carbone, ou
- un radical aryl, comprenant phényl, naphtyl, thiényl, furyl, pyridyl, pyrimidyl, pyridazinyl, pyrazinyl, benzothiényl, benzofuryl, quinolinyl, isoquinolinyl, ou méthylène dioxyphényl, éventuellement substitué par 1 à 3 groupements choisis parmi R⁶,
- R⁵ représente :
- H, sauf quand R⁴ est H, ou
- un radical alkyl, de 1 à 6 atomes de carbone ou
- acyl, de 2 à 6 atomes de carbone ou
- un radical phényl; éventuellement substitué par 1 à 3 groupes parmi R6;
- R⁶ représente :
- un atome d'halogène ou
- un radical alkyl, haloalkyl, alkoxy, haloalkoxy, alkylthio, haloalkylthio ou alkylsulfonyl de 1 à 6 atomes de carbone ou
- un radical cycloalkyl, halocycloalkyl, alcényloxy, alcynyloxy, alcénylthio, alcynylthio de 3 à 6 atomes de carbone ou
- le groupe nitro ou cyano ou
- un radical amino éventuellement mono ou disubstitué par un radical alkyl ou acyl de 1 à 6 atomes de carbone ou alkoxycarbonyl de 2 à 6 atomes de carbone
- un radical phényl, phénoxy ou pyridyloxy, ces radicaux étant éventuellement substitués par 1 à 3 groupements, identiques ou différents, choisis parmi R⁷,
- R⁷ représente :
- un atome d'halogène choisi parmi le fluor, le chlore,le brome, l'iode ou
- un radical alkyle, linéaire ou ramifié, contenant de 1 à 6 atomes de carbone, ou
- un radical alkoxy ou alkylthio, linéaire ou ramifié, contenant de 1 à 6 atomes de carbone ou
- un radical haloalkoxy ou haloalkylthio, linéaire ou ramifié, contenant de 1 à 6 atomes de carbone ou
- un radical nitrile ou
- un radical nitro ;
et les formes salifiées acceptables en agriculture de ces composés.

2. Composés optiquement actifs selon la revendication 1 de formule II :

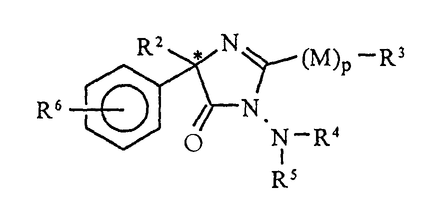

3. Composé selon la revendication 2, **caractérisé en ce qu**'il est l'énantiomère S (+) du composé de formule II, dans laquelle R² est un méthyle, M est un atome de soufre, p égale 1, R³ est un méthyle, R⁴ est un phényle, R⁵ et R⁶ représentent un atome d'hydrogène.

4. Procédé de préparation des composés de formule I dans laquelle p = 1 et M = S, selon l'une des revendications 1 à 3, par réaction du composé de formule III : 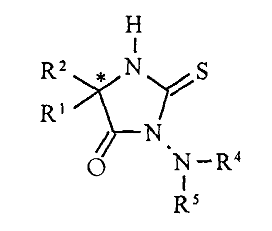 avec le composé de formule R³X, dans laquelle X représente l'atome de chlore, de brome ou d'iode ou le groupe sulfate, ou un groupe alkylsulfonyloxy ou arylsulfonyloxy,
dans un solvant et en présence d'une base, à une température comprise entre -5°C et +80°C.

5. Procédé de préparation selon la revendication 4 **caractérisé en ce que** le solvant est choisi parmi les éthers, les éthers cycliques, les esters d'alkyls, l'acétonitrile, les alcools de 1 à 3 atomes de carbone, les solvants aromatiques, de préférence le tétrahydrofurane.

6. Procédé de préparation selon la revendication 4 **caractérisé en ce que** la base est choisie parmi un alcoolate, de préférence le tertiobutylate de potassium, un hydroxyde alcalin ou alcalino-terreux, un carbonate alcalin ou une amine tertiaire.

7. Procédé de préparation des composés de formule I, dans laquelle p = 1 et M = O, selon l'une des revendications 1 à 3, **caractérisé en ce que** l'on fait réagir le composé de formule I correspondant pour lequel p = 1 et M = S, avec l'alcool de formule R³OH, dans un solvant, en présence d'une base forte, et à une température comprise entre 50 et 80 °C.

8. Procédé de préparation selon la revendication 7 **caractérisé en ce que** la base forte est choisie parmi un hydroxyde alcalin, une base organique forte, ou un alcoolate alcalin de formule R³O⁻Met.⁺, dans laquelle Met.⁺ représente un métal alcalin ou alcalinoterreux.

9. Procédé de préparation selon la revendication 7 **caractérisé en ce que** la réaction est effectuée de en prenant pour solvant l'alccol R³OH et pour base l'alcoolate de sodium R³O⁻Na⁺.

10. Procédé de préparation des composés de formule I, dans laquelle p = 0 et R³ est un atome d'hydrogène, selon l'une des revendications 1 à 3, par réaction du composé de formule VII : 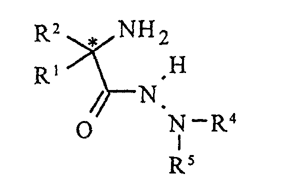 avec un excès du diméthylacétal du diméthylformamide, à une température comprise entre 10 et 100° C.

11. Procédé de préparation des composés optiquement actifs de formule I dans laquelle R³ est un radical C₁-C₂ alkyl, éventuellement halogéné, et dans laquelle p = 0 ou p = 1 et M = CH2 ,selon l'une des revendications 1 à 3, par réaction du composé de formule IX : 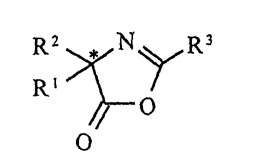 avec le composé de formule V :

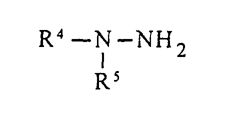

12. Composés optiquement actifs utiles notamment comme intermédiaires dans la préparation des composés de formule I selon l'une des revendications 1 à 3, **caractérisés en ce qu**'ils ont pour formule: 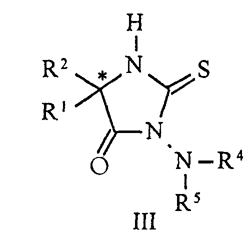 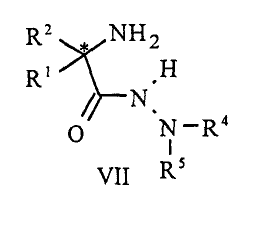 dans lesquelles R¹ à R⁵ ont les mêmes significations que dans la formule générale I de la revendication 1,
et de formule IX : 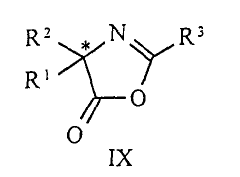 dans laquelle R³ représente un radical C₁-C₃ alkyl éventuellement halogéné,
et de formule XIIb : 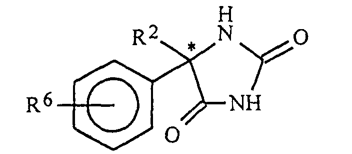 dans laquelle R² a la même signification que dans la revendication 1, R⁶ représente un radical phényl, phénoxy ou pyridyloxy, ces radicaux étant éventuellement substitués par 1 à 3 groupements, identiques ou différents, choisis parmi R⁷ tel que défini dans la revendication 1.

13. Composés selon la revendication 12, **caractérisé en ce que**, dans les formules III, VII, et IX, R1 est un phényle et R2 est un méthyle.

14. Compositions fongicides comprenant, en association avec un ou des supports solides ou liquides acceptables en agriculture et/ou des agens tensio-actifs également acceptables en agriculture, une (ou plusieurs) matière active choisie parmi les composés de formule I, selon l'une des revendications 1 à 3.

15. Procédé de traitement des cultures atteintes ou susceptibles d'être atteintes par les maladies fongiques, **caractérisé en ce que** l'on applique, de façon préventive ou curative, une quantité efficace d'un composé optiquement actif de formule I, selon l'une des revendications 1 à 3.

16. Procédé de traitement des cultures selon la revendication 15 **caractérisé en ce que** les composés de formule I sont appliqués à des doses de 0,005 à 5 kg/ha, de préférence de 0,01 à 1 kg/ha.

## Patentansprüche

1. Optisch aktive 2-Imidazolin-5-on-Derivate der allgemeinen Formel I: 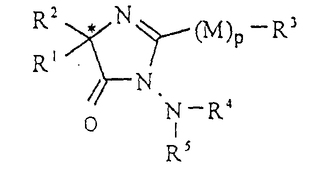 in der:
- M für ein Sauerstoff- oder Schwefelatom oder einen gegebenenfalls halogenierten CH₂-Rest steht;
p eine ganze Zahl gleich 0 oder 1 ist;
- * bezeichnet, daß das asymmetrische Kohlenstoffatom einer stereospezifischen Konfiguration entspricht;
- R¹ und R² unterschiedlich sind und für:
- einen Alkyl- oder Halogenalkylrest mit 1 bis 6 Kohlenstofftomen oder
- einen Alkoxyalkyl-, Alkylthioalkyl-, Alkylsulfonylalkyl-, Monoalkylaminoalkyl, -alkenyl- oder -alkinylrest mit 2 bis 6 Kohlenstoffatomen oder
- einen Dialkylaminoalkyl- oder Cycloalkylrest mit 3 bis 7 Kohlenstoffatomen oder
- einen Arylrest, umfassend Phenyl, Naphthyl, Thienyl, Furyl, Pyridyl, Benzothienyl, Benzofuryl, Chinolinyl, Isochinolinyl oder Methylendioxyphenyl, gegebenenfalls substituiert durch 1 bis 3 Gruppierungen, ausgewählt unter R⁶ oder
- einen Arylalkyl-, Aryloxyalkyl-, Arylthioalkyl- oder Arylsulfonylalkylrest, wobei die Begriffe Aryl und Alkyl die oben angegebenen Definitionen haben,
stehen;
- R¹ und R² mit dem Kohlenstoffatom, über das sie an den Ring gebunden sind, einen Carbocycius oder einen Heterocyclus mit 5 bis 7 Atomen bilden können, wobei diese Ringe mit einem Phenyl gegebenenfalls substituiert durch 1 bis 3 Gruppen, ausgewählt aus R⁶, anneliert sein können,
R³ für:
- ein Wasserstoffatom oder einen C₁-C₂-Alkylrest, der gegebenenfalls halogeniert ist, wenn p gleich 0 ist oder (M)p ein CH₂-Rest ist,
- einen C₁-C₂-Alkylrest, der gegebenenfalls halogeniert ist, wenn (M)p für ein Sauerstoff- oder Schwefelatom steht;
steht;
R⁴ für:
- das Wasserstoffatom oder
- eine Alkylgruppe mit 1 bis 6 Kohlenstoffatomen oder
- einen Arylrest, umfassend Phenyl, Naphthyl, Thienyl, Furyl, Pyridyl, Pyrimidyl, Pyridazinyl, Pyrazinyl, Benzothienyl, Benzofuryl, Chinolinyl, Isochinolinyl oder Methylendioxyphenyl, gegebenenfalls substituiert durch 1 bis 3 Gruppierungen, ausgewählt unter R⁶,
steht;
R⁵ für:
- H, außer R⁴ ist H, oder
- einen Alkylrest mit 1 bis 6 Kohlenstoffatomen oder
- Acylrest mit 2 bis 6 Kohlenstoffatomen oder
- einen Phenylrest, gegebenenfalls substituiert durch 1 bis 3 Gruppen aus R⁶,
steht;
R⁶ für
- ein Halogenatom oder
- einen Alkyl-, Halogenalkyl-, Alkoxy-, Halogenalkoxy-, Alkylthio-, Halogenalkylthio- oder Alkylsulfonylrest mit 1 bis 6 Kohlenstoffatomen oder
- einen Cycloalkyl-, Halogencycloalkyl-, Alkenyloxy-, Alkinyloxy-, Alkenylthio-, Alkinylthiorest mit 3 bis 6 Kohlenstoffatomen oder
- die Nitro- oder Cyangruppe oder
- einen Aminorest, gegebenenfalls mono- oder disubstituiert durch einen Alkyl- oder Acylrest mit 1 bis 6 Kohlenstoffatomen oder Alkoxycarbonylrest mit 2 bis 6 Kohlenstoffatomen,
- einen Phenyl-, Phenoxy- oder Pyridyloxyrest, wobei diese Reste gegebenenfalls durch 1 bis 3 Gruppen, die gleich oder verschieden sind und unter R⁷ ausgewählt sind, substituiert sind,
steht;
- R⁷ für:
- ein Halogenatom, ausgewählt aus Fluor, Chlor, Brom, Iod, oder
- einen linearen oder verzweigten Alkylrest, der 1 bis 6 Kohlenstoffatome enthält, oder
- einen linearen oder verzweigten Alkoxy- oder Alkylthiorest, der 1 bis 6 Kohlenstoffatome enthält, oder
- einen linearen oder verzweigten Halogenalkoxy- oder Halogenalkylthiorest, der 1 bis 6 Kohlenstoffatome enthält, oder
- einen Nitrilrest oder
- einen Nitrorest
steht;
und die in der Landwirtschaft akzeptablen Salzformen dieser Verbindungen.

2. Optisch aktive Verbindungen nach Anspruch 1 mit der Formel II:

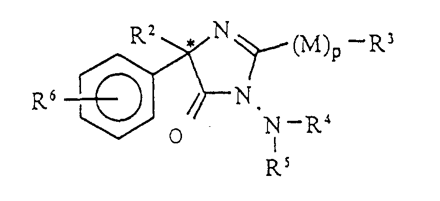

3. Verbindung nach Anspruch 2, **dadurch gekennzeichnet**, daß sie das S (+)-Enantiomer der Verbindung der Formel II ist, in der R² ein Methyl ist, M ein Schwefelatom ist, p gleich 1 ist, R³ ein Methyl ist, R⁴ ein Phenyl ist, R⁵ und R⁶ für ein Wasserstoffatom stehen.

4. Verfahren zur Herstellung der Verbindungen der Formel I, in der p = 1 und M = S, nach einem der Ansprüche 1 bis 3 durch Umsetzung der Verbindung der Formel III: 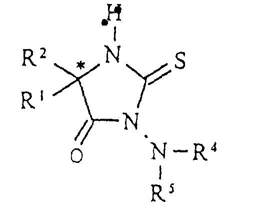 mit der Verbindung der Formel R³X, in der X für das Chlor-, Brom- oder Iodatom oder die Sulfatgruppe oder eine Alkylsulfonyloxy- oder Arylsulfonyloxygruppe steht, in einem Lösemittel und in Gegenwart einer Base bei einer Temperatur zwischen -5°C und +80°C.

5. Herstellungsverfahren nach Anspruch 4, **dadurch gekennzeichnet**, daß das Lösemittel unter den Ethern, den cyclischen Ethern, den Alkylestern, Acetonitril, den Alkoholen mit 1 bis 3 Kohlenstoffatomen, den aromatischen Lösemitteln, vorzugsweise Tetrahydrofuran, ausgewählt wird.

6. Herstellungsverfahren nach Anspruch 4, **dadurch gekennzeichnet**, daß die Base unter einem Alkoholat, vorzugsweise Kalium-tert.-butylat, einem Alkali- oder Erdalkalihydroxid, einem Alkalicarbonat oder einem tertiären Amin ausgewählt wird.

7. Verfahren zur Herstellung der Verbindungen der Formel I, in der p = 1 und M = O, nach einem der Ansprüche 1 bis 3, **dadurch gekennzeichnet**, daß man die entsprechende Verbindung der Formel I, für die p = 1 und M = S, mit dem Alkohol der Formel R³OH in einem Lösemittel in Gegenwart einer starken Base und bei einer Temperatur zwischen 50 und 80°C reagieren läßt.

8. Herstellungsverfahren nach Anspruch 7, **dadurch gekennzeichnet**, daß die starke Base unter einem Alkalihydroxid, einer starken organischen Base oder einem Alkalialkoholat der Formel R³O⁻Met.⁺, in der Met.⁺ für ein Alkali- oder Erdalkalimetall steht, ausgewählt wird.

9. Herstellungsverfahren nach Anspruch 7, **dadurch gekennzeichnet**, daß die Umsetzung ausgeführt wird, indem man als Lösemittel den Alkohol R³OH und als Base Natriumalkoholat R³O⁻Na⁺ nimmt.

10. Verfahren zur Herstellung der Verbindungen der Formel I, in der p = 0 und R³ ein Wasserstoffatom ist, nach einem der Ansprüche 1 bis 3 durch Umsetzung der Verbindung der Formel VII: 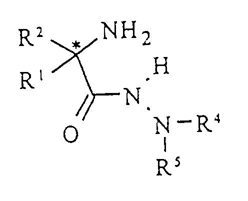 mit einem Überschuß von Dimethylformamiddimethylacetal bei einer Temperatur zwischen 10 und 100°C.

11. Verfahren zur Herstellung der optisch aktiven Verbindungen der Formel I, in der R³ ein gegebenenfalls halogenierter C₁-C₂-Alkylrest ist und in der p = 0 oder p = 1 und M = CH₂, nach einem der Ansprüche 1 bis 3 durch Umsetzung der Verbindung der Formel IX: 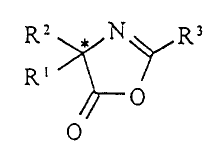 mit der Verbindung der Formel V:

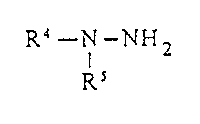

12. Optisch aktive Verbindungen, die insbesondere als Zwischenprodukte bei der Herstellung der Verbindungen der Formel I nach einem der Ansprüche 1 bis 3 nützlich sind, **dadurch gekennzeichnet**, daß sie als Formel: 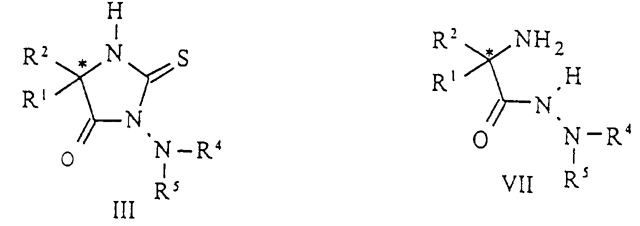 in denen R¹ bis R⁵ die gleichen Bedeutungen wie in der allgemeinen Formel I des Anspruchs 1 haben, haben:
und der Formel IX: 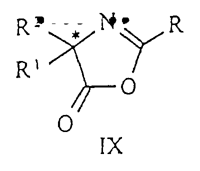 in der R³ für einen gegebenenfalls halogenierten C₁-C₃-Alkylrest steht;
und der Formel XIIb: 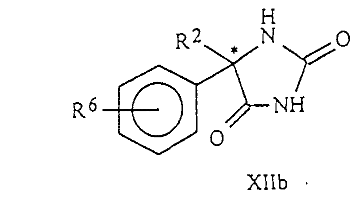 in der R² die gleiche Bedeutung wie in Anspruch 1 hat, R⁶ für einen Phenyl-, Phenoxy- oder Pyridyloxyrest steht, wobei diese Reste gegebenenfalls durch 1 bis 3 Gruppen, die gleich oder verschieden sind und unter R⁷, wie in Anspruch 1 definiert, ausgewählt sind, substituiert sind.

13. Verbindungen nach Anspruch 12, **dadurch gekennzeichnet**, daß in den Formeln III, VII und IX R1 ein Phenyl und R2 ein Methyl ist.

14. Fungizide Zusammensetzungen, die in Verbindung mit einem oder mehreren festen oder flüssigen, in der Landwirtschaft akzeptablen Trägern und/oder gleichfalls in der Landwirtschaft akzeptablen grenzflächenaktiven Mitteln einen (oder mehrere) Wirkstoff, ausgewählt unter den Verbindungen der Formel I nach einem der Ansprüche 1 bis 3, umfassen.

15. Verfahren zur Behandlung von Kulturen, die von Pilzerkrankungen befallen sind oder von Pilzerkrankungen befallen werden können, **dadurch gekennzeichnet**, daß man präventiv oder zur Heilung eine wirksame Menge einer optisch aktiven Verbindung der Formel I nach einem der Ansprüche 1 bis 3 ausbringt.

16. Verfahren zur Behandlung von Kulturen nach Anspruch 15, **dadurch gekennzeichnet**, daß die Verbindungen der Formel I in Dosen von 0,005 bis 5 kg/ha, vorzugsweise 0,01 bis 1 kg/ha ausgebracht werden.

## Claims

1. Optically active 2-imidazolin-5-one derivatives of general formula I 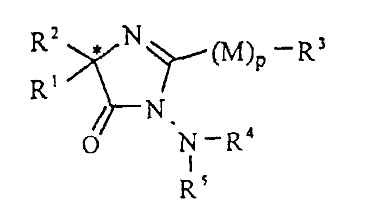 in which:
- M represents an oxygen or sulphur atom, or an optionally halogenated CH₂ radical;
- p is an integer equal to 0 or 1;
- * means the asymmetric carbon atom corresponding to a stereospecific configuration;
- R¹ and R² are different and represent:
- an alkyl or haloalkyl radical containing 1 to 6 carbon atoms or
- an alkoxyalkyl, alkylthioalkyl, alkylsulphonylalkyl, monoalkylaminoalkyl, alkenyl or alkynyl radical containing 2 to 6 carbon atoms or
- a dialkylaminoalkyl or cycloalkyl radical containing 3 to 7 carbon atoms or
- an aryl radical comprising phenyl, naphthyl, thienyl, furyl, pyridyl, benzothienyl, benzofuryl, quinolyl, isoquinolyl or methylenedioxyphenyl, optionally substituted by 1 to 3 groups chosen from R⁶ or
- an arylalkyl, aryloxyalkyl, arylthioalkyl or arylsulphonylalkyl radical, the terms aryl and alkyl having the definitions given above or
- R¹ and R² can form, with the carbon to which they are bonded on the ring, a carbocycle or a heterocycle having from 5 to 7 atoms, it being possible for these rings to be fused to a phenyl, optionally substituted by 1 to 3 groups chosen from R⁶;
- R³ represents:
- a hydrogen or an optionally halogenated C₁-C₂ alkyl radical, when p equals 0 or (M)p is a CH₂ radical,
- an optionally halogenated C₁-C₂ alkyl radical, when (M)p represents an oxygen or sulphur atom;
- R⁴ represents:
- the hydrogen atom or
- an alkyl group containing 1 to 6 carbon atoms or
- an aryl radical, comprising phenyl, naphthyl, thienyl, furyl, pyridyl, pyrimidinyl, pyridazinyl, pyrazinyl, benzothienyl, benzofuryl, quinolyl, isoquinolyl or methylenedioxyphenyl, optionally substituted by 1 to 3 groups chosen from R⁶,
- R⁵ represents:
- H, except when R⁴ is H, or
- an alkyl radical containing 1 to 6 carbon atoms or
- acyl containing 2 to 6 carbon atoms or
- a phenyl radical; optionally substituted by 1 to 3 groups from R⁶;
- R⁶ represents:
- a halogen atom or
- an alkyl, haloalkyl, alkoxy, haloalkoxy, alkylthio, haloalkylthio or alkylsulphonyl radical containing 1 to 6 carbon atoms or
- a cycloalkyl, halocycloalkyl, alkenyloxy, alkynyloxy, alkenylthio or alkynylthio radical containing 3 to 6 carbon atoms or
- the nitro or cyano group or
- an amino radical, optionally mono- or disubstituted by an alkyl or acyl radical containing 1 to 6 carbon atoms or an alkoxycarbonyl radical containing 2 to 6 carbon atoms
- a phenyl, phenoxy or pyridyloxy radical, these radicals optionally being substituted by 1 to 3 identical or different groups chosen from R⁷,
- R⁷ represents:
- a halogen atom chosen from fluorine, chlorine, bromine or iodine or
- a linear or branched alkyl radical containing from 1 to 6 carbon atoms or
- a linear or branched alkoxy or alkylthio radical containing from 1 to 6 carbon atoms or
- a linear or branched haloalkoxy or haloalkylthio radical containing from 1 to 6 carbon atoms or
- a nitrile radical or
- a nitro radical;
and the agriculturally-acceptable salified forms of these compounds.

2. Optically active compounds according to claim 1 of formula II:

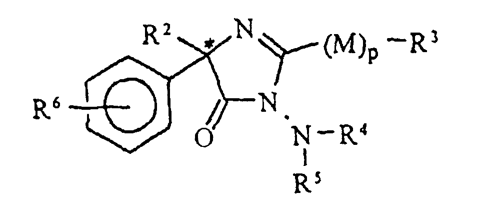

3. Compound according to claim 2, **characterized in that** it is the S-(+) enantiomer of the compound of formula II in which R² is a methyl, M is a sulphur atom, p is equal to 1, R³ is a methyl, R⁴ is a phenyl, and R⁵ and R⁶ represent a hydrogen atom.

4. Process for the preparation of the compounds of formula I in which p = 1 and M = S according to one of claims 1 to 3 by reaction of the compound of formula III: 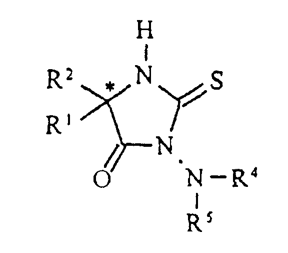 with the compound of formula R³X in which X represents the chlorine, bromine or iodine atom or the sulphate group or an alkylsulphonyloxy or arylsulphonyloxy group,
in a solvent and in the presence of a base, at a temperature of between -5°C and +80°C.

5. Preparation process according to claim 4, **characterized in that** the solvent is chosen from ethers, cyclic ethers, alkyl esters, acetonitrile, alcohols containing 1 to 3 carbon atoms or aromatic solvents, preferably tetrahydrofuran.

6. Preparation process according to claim 4, **characterized in that** the base is chosen from an alkoxide, preferably potassium tert-butoxide, an alkali metal or alkaline-earth metal hydroxide, an alkali metal carbonate or a tertiary amine.

7. Process for the preparation of the compounds of formula I in which p = 1 and M = O according to one of claims 1 to 3, **characterized in that** the corresponding compound of formula I for which p = 1 and M = S is reacted with the alcohol of formula R³OH in a solvent, in the presence of a strong base and at a temperature of between 50 and 80°C.

8. Preparation process according to claim 7, **characterized in that** the strong base is chosen from an alkali metal hydroxide, a strong organic base or an alkali metal alkoxide of formula R³O⁻Met⁺ in which Met⁺ represents an alkali metal or alkaline-earth metal.

9. Preparation process according to claim 7, **characterized in that** the reaction is carried out by taking the alcohol R³OH as solvent and the sodium alkoxide R³O⁻Na⁺ as base.

10. Process for the preparation of the compounds of formula I in which p = 0 and R³ is a hydrogen atom according to one of claims 1 to 3 by reaction of the compound of formula VII: 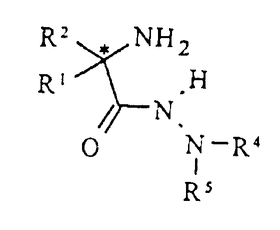 with an excess of dimethylformamide dimethyl acetal at a temperature of between 10 and 100°C.

11. Process for the preparation of the optically active compounds of formula I in which R³ is an optionally halogenated C₁-C₂ alkyl radical and in which p = 0 or p = 1 and M = CH₂ according to one of claims 1 to 3 by reaction of the compound of formula IX: 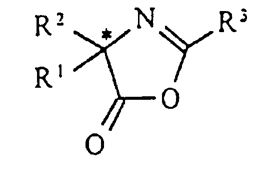 with the compound of formula V:

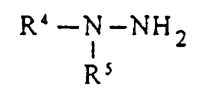

12. Optically active compounds which are useful especially as intermediates in the preparation of the compounds of formula I according to one of claims 1 to 3, **characterized in that** they have the formula: 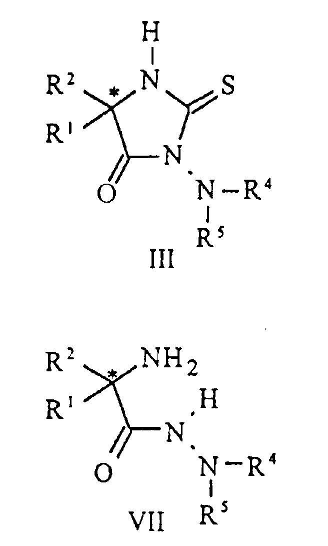 in which R¹ to R⁵ have the same meanings as in the general formula I of claim 1,
and of formula IX: 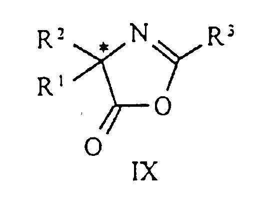 in which R³ represents an optionally halogenated C₁-C₃ alkyl radical,
and of formula XIIb: 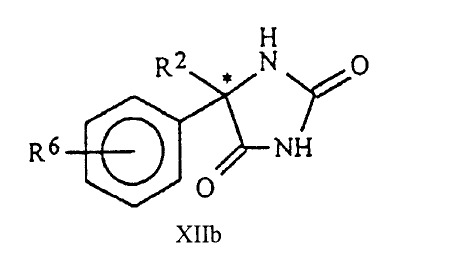 in which R² has the same meaning as in claim 1 and R⁶ represents a phenyl, phenoxy or pyridyloxy radical, these radicals optionally being substituted by 1 to 3 identical or different groups chosen from R⁷ as defined in claim 1.

13. Compounds according to claim 12, **characterized in that**, in the formulae III, VII and IX, R¹ is a phenyl and R² is a methyl.

14. Fungicidal compositions comprising, in combination with one or more solid or liquid vehicles which are acceptable in agriculture and/or surface-active agents which are also acceptable in agriculture, one (or a number of) active material chosen from the compounds of formula I according to one of claims 1 to 3.

15. Process for the treatment of crops affected by or capable of being affected by fungal diseases, **characterized in that** an effective amount of an optically active compound of formula I according to one of claims 1 to 3 is applied preventively or curatively.

16. Process for the treatment of crops according to claim 15, **characterized in that** the compounds of formula I are applied at doses of 0.005 to 5 kg/ha, preferably of 0.01 to 1 kg/ha.
